# EUROPEAN PATENT APPLICATION

(11) **EP 4 338 732 A1**
(43) Date of publication of application: **20.03.2024**
(21) Application number: 22198179.8
(22) Date of filing: 27.09.2022
(51) Int. Cl.: A61K 31/215, A61P 31/04

(54) **LEFAMULIN AND ITS DERIVATIVES FOR USE IN THE TREATMENT OF TULAREMIA**

(30) Priority: 16.09.2022 US 202263407352 P
(71) Applicant: Nabriva Therapeutics GMBH, 1110 Wien (AT)
(72) Inventor: The designation of the inventor has not yet been filed
(74) Representative: Schwarz & Partner Patentanwälte GmbH

(57) **Abstract**

A compound of formula (I) 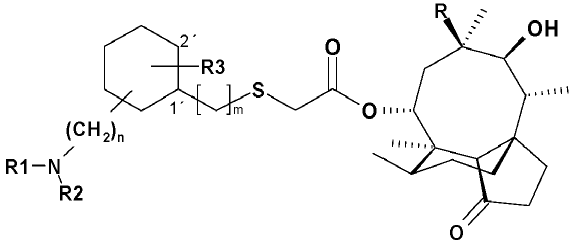 wherein
n is 0 to 4;
m is 0 or 1 with the proviso that the sulphur atom and R₃ are in vicinal position (if m = 0 then R₃ is in position 2', and if m = 1 then R₃ is on position 1');
R is ethyl or vinyl;
R₁ is hydrogen or (C₁₋₆)alkyl,
R₂ is hydrogen or
- (C₃₋₆)cycloalkyl, or
- unsubstituted (C₁₋₆)alkyl, or
- (C₁₋₆)alkyl substituted by one or more of
- hydroxy; preferably one or two,
- methoxy,
- halogen, and
- (C₃₋₆)cycloalkyl, or

R₁ and R₂ together with the nitrogen atom to which they are attached form a 5 to 7 membered heterocyclic ring containing at least 1 nitrogen atom or 1 nitrogen and 1 additional heteroatom e. g. selected from N or O, or
R₁ is hydroxy and R₂ is formyl;
R₃ is OH, OR₄, a halogen atom, or
R₃ is bound to 2' and represents -O-(CH₂)ₚ-O- with p is 2 or 3;
R₄ is unsubstituted (C₁₋₆)alkyl or (C₃₋₆)cycloalkyl,
or a pharmaceutically acceptable salt, solvate, prodrug or metabolite thereof
for the specific use in the treatment or prevention of a bacterial infection mediated by F. *tularensis.*

## Description

The present invention relates to a novel therapeutic use of Pleuromutilins.

Pleuromutilin, a compound of formula (A) 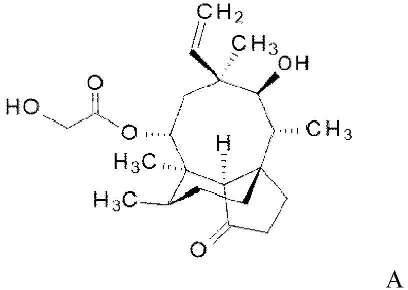 is a naturally occurring antibiotic, produced e.g. by the basidiomycetes *Pleurotus mutilus* and *P. passeckerianus,* see e.g. The Merck Index, 12th edition, item 7694.

A number of further Pleuromutilins having the principle ring structure of Pleuromutilin and being substituted at the primary hydroxy group have been developed, e.g. as antibacterials. Due to their pronounced antibacterial activity, a group of Pleuromutilin derivatives, amino-hydroxy-substituted cyclohexylsulfanylacetylmutilins, as disclosed in WO 2008/113089, have been found to be of particular interest. As described in WO 2008/113089 14-O-{[(4-Amino-2-hydroxy-cyclohexyl)-sulfanyl]-acetyl}-mutilins are particularly useful compounds because of their activity against Gram-positive and Gram-negative bacteria.

Pharmaceutical active compounds derived from Pleuromutilin (semi synthetic compounds) are inhibitors of ribosomal protein synthesis in bacteria. Representatives of semisynthetic Pleuromutilins for human use are Retapamulin (compound of formula (B), approved as AltargoP^{®}, AltabaxP^{®}), a topical agent approved for short term treatment of impetigo and infected small lacerations, abrasions or sutured wounds, and Lefamulin (compound of formula (VII), approved as Xenleta^{®}) for the treatment of adults with community-acquired bacterial pneumonia (CABP). Valnemulin (compound of formula (C), approved as Econor^{®}) and Tiamulin (compound of formula (D), approved as Denagard^{®}) are two other semi-synthetic Pleuromutilin derivatives which have been used systemically as antibiotics in veterinary medicine for many years.

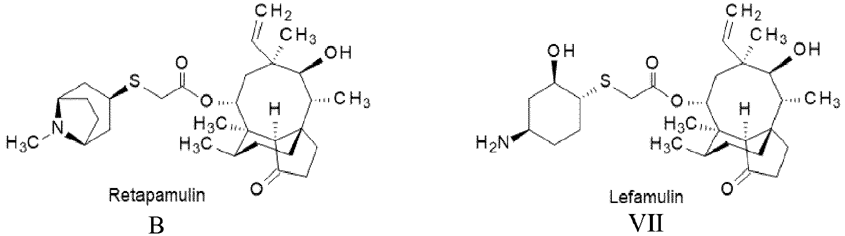

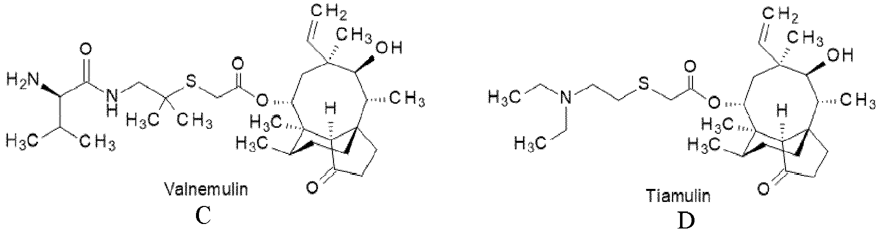

Approved semisynthetic compounds derived from Pleuromutilin have shown excellent activity against bacterial organisms which include inter alia *Streptococcus pneumoniae, Haemophilus influenzae, Staphylococcus aureus* (including MRSA), *Moraxella catarrhalis, Legionella pneumophila, Chlamydophila pneumoniae* and *Mycoplasma pneumoniae.*

Tularemia is an infectious disease caused by the bacterium *Francisella tularensis* (*F. tularensis*)*.* The infectious disease of human is zoonotic as the pathogen circulates in wild animal hosts transmitted for example by parasites. The frequency of infections with *F*. *tularensis* in humans is rather low (rare disease) and the occurrence characterized by local outbreaks. Tularemia may result from aerosolized *F*. *tularensis* and the bacterium is highly infective. Therefore, the causative agent, the bacterium *Francisella tularensis,* is regarded as a viable biological warfare agent, i.e. is regarded as a biothreat.

WO 2007/062333, WO 2007/062334, and WO 2007/062335 refer to specific pleuromutilin compounds with an O-acyl-carbamate linker and mention activity of individual compounds against several bacteria including *F*. *tularensis.* However, the application discloses no microbiological data such as minimum inhibitory concentration (MIC).

### SUMMARY OF THE INVENTION

Surprisingly, it has now been found that the Pleuromutilin derivatives disclosed in WO 2008/113089A1 show antimicrobial activity against the biothreat bacteria *F*. *tularensis.* The Pleuromutilin derivatives, in particular Lefamulin, are active *in vitro* and *in vivo* against strains of the bacterium known to cause tularemia in humans.

Therefore, in a first aspect, the present invention relates to a compound as defined in claims 1 to 6, in particular Lefamulin, or any pharmaceutically acceptable salt, solvate, ester of metabolite thereof, for the specific use in the treatment or prevention of a bacterial infection mediated by *F*. *tularensis.*

In a further aspect, the present invention relates to a method of treatment or prevention of a bacterial infection mediated by *F*. *tularensis* comprising administering a compound as defined in any of claims 1 to 6, in particular Lefamulin, or a pharmaceutically acceptable salt, solvate, ester of metabolite thereof to a subject in need of such treatment.

### BRIEF DESCRIPTION OF THE DRAWINGS

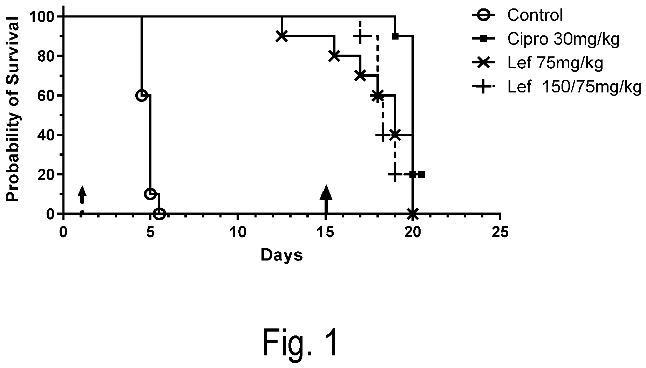

Fig. 1 shows the probability of survival over time as a result of an *in vivo* study with mice challenged with an aerosol of *F*. *tularensis* subsp. *tularensis* SchuS4 for Lefamulin (Lef) at different oral dose regimes compared with the untreated control and treatment with ciprofloxacin (cipro).

### DETAILED DESCRIPTION OF THE INVENTION

Lefamulin is the INN for a compound of generic formula (I), more particular, Lefamulin is a compound of formula (VII) 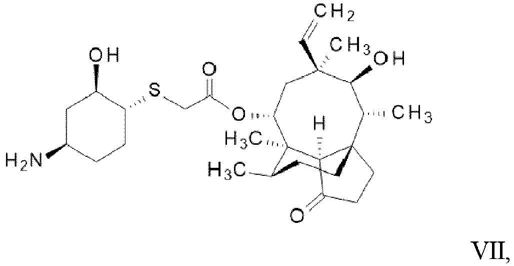 i.e. 14-O-{[(1*R*, 2*R*, 4*R*)-4-amino-2-hydroxy-cyclohexylsulfanyl]-acetyl}-mutilin (also known as "BC-3781").

In the following, the term "Lefamulin", when generally used without additional explanation, is intended to encompass both Lefamulin in free base form, as well as its salts and solvates.

Lefamulin has been developed for systemic use to treat serious bacterial infections in humans and was approved for medical use in the United States in 2019 to treat adults with community-acquired bacterial pneumonia (CABP).

It has been found that the compounds used according to the present invention have an antibacterial effect on *F*. *tularensis.* As shown in Example 1 Lefamulin shows potent inhibition against various different subspecies of *F*. *tularensis.* The *in vitro* activity translates into an *in vivo* effect as the oral treatment with Lefamulin was shown to be effective in prolonging the survival after aerosol challenge with the highly virulent SchuS4 strain of *F*. *tularensis* subsp. *tularensis* (Example 2).

Accordingly, the present invention concerns the compound as defined in claims 1 to 6, especially Lefamulin, for use in the treatment or prevention of a bacterial infection mediated by *F*. *tularensis,* the use of said compound in the treatment or prevention of a bacterial infection mediated by *F*. *tularensis* and/or the use of said compound in the manufacture of a medicament for the treatment or prevention of a bacterial infection mediated by *F*. *tularensis.*

*Francisella tularensis* is a Gram-negative, facultative intracellular bacterium, able to survive and grow within eukaryotic cells including macrophages. *Francisella tularensis* is currently divided into four subspecies (abbreviated as subsp. / ssp.):
- *tularensis* (Type A or serovar A)
- *holarctica* (Type B or serovar B)
- *novicida,* and
- *mediasiatica.*

Type A strains are more virulent and found exclusively in North America and Mexico and cause the most severe form of human disease. Type B is found throughout the Northern Hemisphere and is less pathogenic in humans than Type A and *F*. *tularensis* subsp. *mediasiatica* found in Central Asia (Champion MD et al., PLoS Pathog. 2009, 5(5): e1000459, DOI: 10.1371/journal.ppat. 1000459). Debate continues on whether subsp. *novicida* is a fourth subspecies of *F*. *tularensis* or a separate species and is sometimes classified as a separate species "*F*. *novicida"* due to its aquatic reservoir and very low virulence in humans (Kingry LC, Petersen JM, Front Cell Infect Microbiol 2014, 4:35, DOI: 10.3389/fcimb.2014.00035; Caspar Y, Maurin M, Front Cell Infect Microbiol 2017, 7:122 DOI: 10.3389/fcimb.2017.00122).

In one embodiment, the bacterial infection is mediated by a subspecies of *F*. *tularensis* selected from the group of *F*. *tularensis* subsp. *tularensis* (Type A), *F*. *tularensis* subsp. *holarctica* (Type B), *F*. *tularensis* subsp. *mediasiatica* and *F*. *tularensis* subsp. *novicida,* preferably a subspecies of *F*. *tularensis* selected from the group of *F*. *tularensis* subsp. *tularensis* (Type A), *F*. *tularensis* subsp. *holarctica* (Type B), and *F*. *tularensis* subsp. *novicida.*

Tularemia is the common term for an infectious disease, i.e. a bacterial infection mediated by *F*. *tularensis.* Depending on the infected subject or the zoonotic origin, non-medical synonyms to tularemia such as rabbit fever or deer fly fever are used.

In one embodiment, the bacterial infection mediated by *F*. *tularensis* is tularemia.

In one embodiment, the compound is administered (or configured for being administered) to a human.

Treating, treatment or to treat as understood herein includes on one hand the complete curing, curation or to cure a condition (the bacterial infection) such that it comes to its end and on the other hand also ameliorating, amelioration or to ameliorate a condition such that its symptoms are reduced at least partially or individually.

Treatment typically includes administering a compound as used according to the present invention to a subject in need thereof, e.g. in one embodiment, a subject being diagnosed to have a bacterial infection mediated by *F*. *tularensis.* The subject may have a medical history including earlier symptoms of the bacterial infection, i.e. resulting in a bacterial infection being diagnosed earlier and the current symptoms occurring later. Accordingly, in a preferred embodiment, the compound for use according to the present invention is administered to a subject showing symptoms of or being diagnosed with a bacterial infection mediated *F*. *tularensis.* However, the invention is useful both in the context of curative or prophylactic treatment.

Preventing, prevention, or to prevent includes administering a compound before a condition is diagnosed or before onset of (all) disease symptoms of the condition. The term prevention is synonymous to prophylaxis.

For example, prevention according to the present invention may be considered after a subject has been infected with *F*. *tularensis,* but has not shown any symptoms of a bacterial infection (asymptomatic carrier) or, wherein a subject has been exposed and/or is prone to exposition to the bacteria.

Accordingly, in one embodiment, the compound is used for post-exposure prophylaxis (PEP).

The appropriate dosage of the compound to be administered according to the present invention, in particular Lefamulin, will, of course, vary depending upon, for example, the individual host, the mode of administration and the nature and severity of the conditions being treated. However, in general, for satisfactory results in larger mammals, for example humans, an indicated daily dosage is in the range from about 0.5 mg to 3 g of a compound used according to the present invention conveniently administered, for example, in divided doses up to four times a day.

The compound used according to the present invention may be administered by any conventional route, for example enterally, e.g. including nasal, buccal, rectal, oral administration; parenterally, e.g. including intravenous, intramuscular, subcutaneous, transdermal administration; or topically, e.g. including pulmonary, epicutaneous, intranasal, intratracheal, dermal administration, e.g. in form of coated or uncoated tablets, capsules, injectable solutions or suspensions, e.g. in the form of ampoules, vials, in the form of ointments, creams, gels, pastes, inhaler powder, foams, tinctures, lip sticks, drops, sprays, patches, or in the form of suppositories. In particular, route and form of administration may be selected in analogous manner to antibiotic agents such as e.g. tetracyclines (e.g. doxycycline, tetracycline), aminoglycosides (e.g. streptomycin, gentamicin), fluoroquinolones (e.g. ciprofloxacin), or chloramphenicol.

Preferably, the compound used according to the present invention is administered intravenously (IV) or orally.

In particular, the oral treatment option is a benefit for broad use in population, for example, where a post-exposure prophylaxis is needed, and the *in vivo* data show an effect for Lefamulin being applied orally.

Preferred pharmaceutical compositions of Lefamulin for injection are disclosed in WO 2016/202788 A1 the contents of which are incorporated herein by reference.

The compound used according to the present invention, in particular Lefamulin, may be administered in the form of a pharmaceutically acceptable salt, e.g. an acid addition salt, or in free form, optionally in the form of a solvate.

In one embodiment, the compound is in the form of a salt and/or a solvate.

A salt of a compound used according to the present invention includes an acid addition salt. Pharmaceutically acceptable acid addition salts include salts of a compound used according to the present invention with an acid, e.g. hydrogen fumaric acid, fumaric acid, tartaric acid, ethane-1,2-disulphonic acid, maleic acid, naphthalin-1,5-sulphonic acid, acetic acid, malic acid, lactic acid, i.e., L-lactic acid, succinic acid, salicylic acid, azelaic acid, 2-[(2,6-dichlorophenyl)amino]benzene acetic acid, hydrochloric acid, deuterochloric acid, and citric acid, preferably hydrochloric acid, acetic acid, L-lactic acid and maleic acid. In a further embodiment, the compound of the invention may be used as acid addition salt with itaconic acid, e.g. Lefamulin itaconate salt (WO 2021/209174).

In a preferred embodiment, the compound used according to the invention is Lefamulin in the form as Lefamulin acetate salt.

Preferred crystalline forms of Lefamulin as well as crystalline salt forms of Lefamulin are disclosed in WO 2011/146954 A1, the contents of which are incorporated herein by reference. Of these, the acetate salt of Lefamulin in crystalline Form B as disclosed in WO 2011/146954 A1 is especially preferred.

The compound used according to the present invention, in particular Lefamulin, may be used for the pharmaceutical treatment contemplated herein alone or in combination with one or more other pharmaceutically active agents. Such other pharmaceutically active agents include those used in the standard-of-care therapy of infections caused by *F*. *tularensis,* e.g. antibacterial compounds such as e.g. tetracyclines (e.g. doxycycline, tetracycline), aminoglycosides (e.g. streptomycin, gentamicin), fluoroquinolones (e.g. ciprofloxacin), or chloramphenicol.

Combinations include fixed combinations, in which two or more pharmaceutically active agents are in the same formulation; kits, in which two or more pharmaceutically active agents in separate formulations are sold in the same package, e.g. with instruction for coadministration; and free combinations in which the pharmaceutically active agents are packaged separately, but instruction for simultaneous or sequential administration are given.

A pharmaceutical composition comprising a compound used according to the present invention, in particular Lefamulin may in addition comprise at least one pharmaceutically acceptable excipient, e.g. carrier or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or buffers.

Such pharmaceutical compositions may be manufactured according, e.g. analogously, to a method as conventional, e.g. by mixing, granulating, coating, dissolving, spray drying, or lyophilizing processes. Unit dosage form may contain, for example, from about 0.5 mg to about 3000 mg, such as 10 mg to about 600 mg.

### Examples

Herein, including the examples, the following abbreviations are used:
- ATCC: American Type Culture Collection
- CAMHB: Cation-adjusted Mueller-Hinton Broth
- CFU: colony forming units
- CLSI: Clinical and Laboratory Standards Institute
- MIC: Minimal Inhibitory Concentration [µg/mL]
- MIC₅₀: MIC required to inhibit growth of 50% of microorganisms
- MIC₉₀: MIC required to inhibit growth of 90% of microorganisms
- SD: standard deviation
- ssp./subsp.: subspecies

### Example 1 - In vitro antibacterial activity (MIC)

**Objective:** Antimicrobial susceptibility testing of Lefamulin (BC-3781) and licensed reference compounds following CLSI standards against a diversity set of *Francisella tularensis* including the subspecies *Francisella tularensis* ssp. *tularensis, F*. *tularensis* ssp. *holarctica* and *F*. *tularensis* ssp. *novicida.*

**Methodology:** The minimum inhibitory concentration (MIC) was determined by the United States Army Medical Research Institute of Infectious Diseases (USAMRIID) against n=15 *F*. *tularensis* isolates for Lefamulin (BC-3781) for each individual isolate following the CLSI guidelines (CLSI Methods for Dilution Antimicrobial Susceptibility Tests for Bacteria That Grow Aerobically; Approved Standard - Ninth Edition. CLSI document M07-A9, ISBN 1-56238-783-9, 2012; CLSI Performance Standards for Antimicrobial Susceptibility Testing; Eighteenth Informational Supplement. CLSI Document M100-S18, ISBN 1-56238-653-0, 2008; Johansson A. et al., J. Bacteriol., 2004, 186:5808-5818, DOI: 10.1128/JB.186.17.5808-5818.2004). Antibiotics were serially diluted two-fold in 50 µL of CAMHB. For all steps with *F*. *tularensis,* CAMHB was supplemented with 2% Isovitalex (Becton Dickinson). The antibiotic ranges were from 0.0039 to 8 µg/mL (ciprofloxacin) or from 0.031 to 64 µg/mL (Lefamulin, erythromycin) based on a final well volume of 100 µL after inoculation.

Plates were incubated at 35°C for 48 h. MICs were determined both by visual inspection and spectrophotometry. When there was a discrepancy between the visual and spectrophotometric MIC, the visual MIC was used in the report and data analysis. All compounds were tested with the recommended CLSI reference quality control strains, *Escherichia coli* ATCC 25922, *Staphylococcus aureus* ATCC 29213, and *Pseudomonas aeruginosa* ATCC 27853 (data not shown).

The MIC of antibiotic that result in 90% inhibition of bacterial growth (MIC₉₀) and 50% inhibition (MIC₅₀) were identified for each compound against the entire diversity set and the subspecies.

**Results:** Table 1 summarizes the *in vitro* antibacterial activity of Lefamulin, erythromycin and ciprofloxacin.

**Table 1. MIC₅₀ and MIC₉₀ (µg/mL) of Lefamulin, erythromycin and ciprofloxacin against F. tularensis including subsp. tularensis (Type A), F. tularensis subsp. holarctica (Type B) and F. tularensis subs. novicida.**

| **Subspecies / Data** | **Lefamulin** | **Erythromycin** | **Ciprofloxacin** |
|---|---|---|---|
| ***F. tularensis,* total** | | | |
| N | 14 | 14 | 14 |
| MIC₅₀ (µg/mL) | 1 | 2 | 0.016 |
| MIC₉₀ (µg/mL | 4 | ≥8 | 0.03 |
| Range | 0.5 - 8 | 0.5 - ≥8 | 0.016-0.5 |

| ***F. tularensis* subsp. *tularensis*** | | | |
|---|---|---|---|
| N | 8 | 8 | 8 |
| MIC₅₀ (µg/mL) | 1 | 1 | 0.016 |
| MIC₉₀ (µg/mL | 1 | 2 | 0.03 |
| Range | 0.5 - 4 | 0.5 - 8 | 0.016 - 0.03 |
| Strain SchuS4 ^{b} | 1 | 2 | 0.016 |

| ***F. tularensis* subsp. *holarctica*** | | | |
|---|---|---|---|
| N ^{a} | 5 | 5 | 5 |
| MIC₅₀ (µg/mL) | 4 | 8 | 0.03 |
| Range | 0.5-8 | 4 - ≥8 | 0.016 - 0.5 |

| ***F. tularensis* subsp. *novicida*** | | | |
|---|---|---|---|
| N | 1 | 1 | 1 |
| MIC | 2 | ≥8 | 0.016 |
| ^{a} non-virulent vaccine strain excluded | | | |
| ^{b} F. *tularensis* subsp. *tularensis* Strain SchuS4 and used to cause tularemia in *in vivo* mouse model of Example 2 | | | |

Lefamulin demonstrated potent *in vitro* activity against the *F*. *tularensis* diversity set (n=14) with MICs ranging between 0.5 and 8 µg/mL. Against *F*. *tularensis* subsp. *tularensis* (Type A; n=8), which is the most virulent subspecies within the species of *F*. *tularensis,* is found primarily in North America and Mexico and which causes the most severe form of human disease, Lefamulin displayed surprisingly potent activity with MIC_{50/90} values of 1 and 1 µg/mL, respectively. All subsp. *tularensis* isolates were inhibited at Lefamulin concentrations of ≤4 µg/mL, including an MIC of 1 µg/mL against the Schu4 strain, the strain that is used to establish tularemia *in vivo* in animals (e.g. mice). Erythromycin displayed slightly weaker activity with MIC_{50/90} values of 1 µg/mL and 2 µg/mL. Ciprofloxacin demonstrated MIC_{50/90} values of 0.016 µg/mL and 0.03 µg/mL against subsp. *tularensis.*

Against *F*. *tularensis* subsp. *holarctica* (Type B; n=5) Lefamulin displayed MIC values ranging from 0.5 to 8 µg/mL and an MIC₅₀ of 4 µg/mL. Similar to erythromycin (range of 4 to ≥8 µg/mL, MIC₅₀ of 8 µg/mL), the Lefamulin MIC values were slightly higher for these less virulent Type B strains than against the *F*. *tularensis* subsp. *tularensis* (Type A). Ciprofloxacin was equally potent against the subsp. *tularensis* and subsp. *holarctica* isolates.

Potent activity was also shown against subsp. *novicida* for Lefamulin (MIC of 2 µg/mL) and ciprofloxacin (MIC of 0.016 µg/mL), whereas erythromycin was inactive (MIC of ≥8 µg/mL).

### Example 2 - Evaluation of Lefamulin Dose to Survival in Mice Following Aerosol Exposure to F. tularensis

**Objective:** This study evaluated the efficacy of Lefamulin when administered at different doses post-aerosol challenge mice with clinically relevant *F*. *tularensis* subsp. *tularensis* strain SchuS4.

### Methodology:

Animals were aerosol challenged with the highly virulent SchuS4 strain of *F*. *tularensis* subsp. *tularensis* (ITI3004). Aerosol bacterial concentrations were serially diluted and plated. The actual challenge dose was 76 times the dose required to kill half the members of a tested population (LD₅₀). At 24 hours post-challenge (PC), administration of negative control (vehicle), ciprofloxacin control, and Lefamulin commenced (onset of the treatment). Doses were given by oral gavage in a 0.1 to 0.2 mL volume (dependent on solubility/formulation). Lefamulin was diluted in water. Doses and treatment schedule are summarized in Table 2 below. All groups were dosed for a total of 14 days followed by an additional 30 days of observation (45 days total post-challenge). The study includes two different dosing regimens for Lefamulin, the 75 mg/kg Lefamulin arm and the 150/75 mg/kg Lefamulin and respectively, wherein the latter includes higher initial dosages at day 1 (loading dose).

**Table 2: Treatment and dose regimes investigated in the in vivo model**

| **Treatment** | **No. of mice/ group** | **Dose route** | **Dose (mg/kg)** | **Dosing Schedule** | **Total daily dose (mg/kg)** |
|---|---|---|---|---|---|
| Vehicle (control) | 10 | oral | 0 | every 6 h | 0 |
| Ciprofloxacin (cipro) | 10 | oral | 30 | every 12 h | 60 |
| Lefamulin (Lef 75 mg/kg) | 10 | oral | 75 | every 12 h | 150 |
| Lefamulin (Lef 150/75 mg/kg) | 10 | oral | 150 (loading dose at day 1)/ 75 (maintenance dose) | every 12 h | 300/ 150 |

### Endpoints:

Survival was assessed four times per day for the first 15 days of the study and twice daily thereafter. At 45 days post-challenge, all remaining surviving animals were euthanized for tissue load determinations. Animals were euthanized by carbon dioxide (CO₂); lungs and spleens were removed, weighed and homogenized in 1 mL sterile saline. Homogenates were serially diluted 1:10 and dilutions (100 µL) plated in duplicate and incubated at 35°C for 48 hours to determine bacterial load (note: limit of bacterial detection is 5 CFU/mL of spleen and lung).

### Results:

Efficacy-survival results are summarized in Figure 1, wherein the small arrow indicates the start of treatment and the larger arrow indicates the end of treatment.

Lefamulin survival was significant for all dose groups (p<0.0008) when compared to the vehicle control (water for injection). While 10 of 10 animals (100%) of the vehicle control group died by Day 5, 100% of animals in the 150/75 mg/kg Lefamulin arm and the ciprofloxacin arm as well as 90% of the 75 mg/kg Lefamulin arm survived by the end of treatment (Day 15). All treatment groups, including both Lefamulin treatment groups and the ciprofloxacin control, succumbed to disease after treatment was terminated with almost 100% mortality 5 days after the last treatment.

In the animals surviving until day 45, the bacterial load was determined. The average counts for the lungs and spleens from the two remaining ciprofloxacin-treated mice were of 6.25×10⁵ CFUs/g and 4×10⁶ CFU/g tissue, respectively. No ciprofloxacin resistance was detected. The counts for lung and spleen in the one remaining Lefamulin-treated animal were 1.3×10⁶ CFU/g and 7.7×10⁶ CFU/g. No resistance was detected against Lefamulin.

The data from this study indicate that Lefamulin and ciprofloxacin showed comparable efficacy against a *F*. *tularensis* infection in the murine *in vivo* model.

## Claims

1. A compound of formula (I) 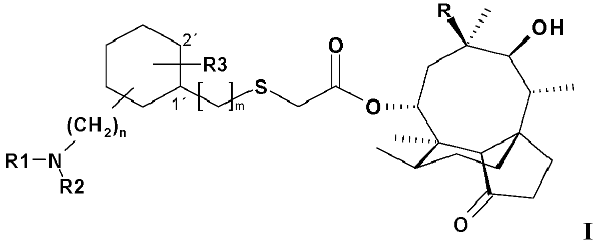 wherein
n is 0 to 4;
m is 0 or 1 with the proviso that the sulphur atom and R₃ are in vicinal position (if m = 0 then R₃ is in position 2', and if m = 1 then R₃ is on position 1');
R is ethyl or vinyl;
R₁ is hydrogen or (C₁₋₆)alkyl,
R₂ is hydrogen or
- (C₃₋₆)cycloalkyl, or
- unsubstituted (C₁₋₆)alkyl, or
- (C₁₋₆)alkyl substituted by one or more of
- hydroxy; preferably one or two,
- methoxy,
- halogen, and
- (C₃₋₆)cycloalkyl, or
R₁ and R₂ together with the nitrogen atom to which they are attached form a 5 to 7 membered heterocyclic ring containing at least 1 nitrogen atom or 1 nitrogen and 1 additional heteroatom e. g. selected from N or O, or
R₁ is hydroxy and R₂ is formyl;
R₃ is OH, OR₄, a halogen atom, or
R₃ is bound to 2' and represents -O-(CH₂)ₚ-O- with p is 2 or 3;
R₄ is unsubstituted (C₁₋₆)alkyl or (C₃₋₆)cycloalkyl,
or a pharmaceutically acceptable salt, solvate, prodrug or metabolite thereof
for the specific use in the treatment or prevention of a bacterial infection mediated by *F*. *tularensis.*

2. Compound for use according to claim 1, wherein the compound is selected from the group consisting of the compounds of formula (II), (III), (IV), (V) and (VI) 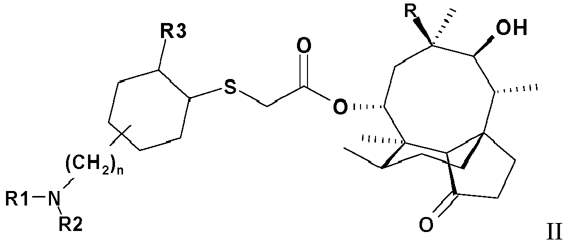 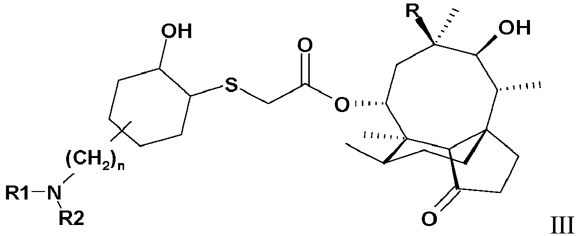 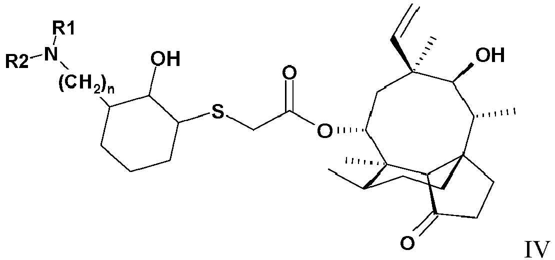 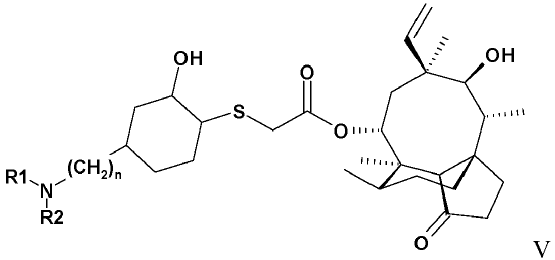 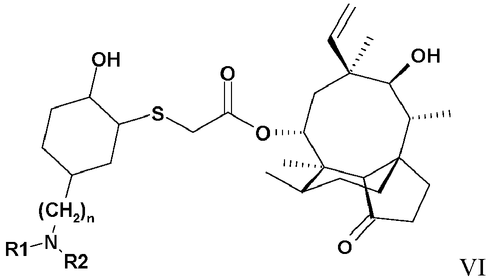 wherein in each case n, R₁ and R₂ are as defined in claim 1.

3. A compound for use according to claim 1 or 2, wherein the compound is selected from the group consisting of
14-O-{[(1R, 2R, 4R)-4-Amino-2-hydroxy-cyclohexylsulfanyl]-acetyl}-mutilin
14-O-{[(1S, 2S, 4S)-4-Amino-2-hydroxy-cyclohexylsulfanyl]-acetyl}-mutilin
14-O-{[(1R, 2R, 5S)-5-Amino-2-hydroxy-cyclohexylsulfanyl]-acetyl}-mutilin
14-O-{[(1S, 2S, 5R)-5-Amino-2-hydroxy-cyclohexylsulfanyl]-acetyl}-mutilin
14-O-{[(1R, 2R, 4S)-4-Amino-2-hydroxy-cyclohexylsulfanyl]-acetyl}-mutilin and the (1S, 2S, 4R) diastereomer thereof
14-O-{[(1R, 2R, 5R)-5-Amino-2-hydroxy-cyclohexylsulfanyl]-acetyl}-mutilin
14-O-{[(1S, 2S, 5S)-5-Amino-2-hydroxy-cyclohexylsulfanyl]-acetyl}-mutilin
14-O-{[(1R, 2R, 3R)-3-Amino-2-hydroxy-cyclohexylsulfanyl]-acetyl}-mutilin and the (1S, 2S, 3S) diastereomer thereof
14-O-{[(1R, 2R, 4R)-4-Diethylamino-2-hydroxy-cyclohexylsulfanyl]-acetyl}-mutilin and the (1S, 2S, 4S) diastereomer thereof
14-O-{[(1R, 2R, 4R)-4-Ethylamino-2-hydroxy-cyclohexylsulfanyl]-acetyl}-mutilin and the (1S, 2S, 4S) diastereomer thereof
14-O-{[(1R, 2R, 5S)-5-Ethylamino-2-hydroxy-cyclohexylsulfanyl]-acetyl}-mutilin and the (1S, 2S, 5R) diastereomer thereof
14-O-{[(1R, 2R, 5S)-5-Diethylamino-2-hydroxy-cyclohexylsulfanyl]-acetyl}-mutilin and the (1S, 2S, 5R) diastereomer thereof
14-O-{[(1R, 2R, 4S)-4-Diethylamino-2-hydroxy-cyclohexylsulfanyl]-acetyl}-mutilin and the (1S, 2S, 4R) diastereomer thereof
14-O-{[(1R, 2R, 5R)-5-Diethylamino-2-hydroxy-cyclohexylsulfanyl]-acetyl}-mutilin and the (1S, 2S, 5S) diastereomer thereof
14-O-{[(1R, 2R, 3R)-3-Ethylamino-2-hydroxy-cyclohexylsulfanyl]-acetyl}-mutilin and the (1S, 2S, 3S) diastereomer thereof
14-O-{[(1R, 2R, 3R)-3-Diethylamino-2-hydroxy-cyclohexylsulfanyl]-acetyl}-mutilin and the (1S, 2S, 3S) diastereomer thereof
14-O-{[(1R, 2R, 4S)-4-(Formyl-hydroxy-amino)-2-hydroxy-cyclohexylsulfanyl]-acetyl}-mutilin and the (1S, 2S, 4R) diastereomer thereof
14-O-{[(1R, 2R, 5S)-5-(Formyl-hydroxy-amino)-2-hydroxy-cyclohexylsulfanyl]-acetyl}-mutilin and the (1S, 2S, 5R) diastereomer thereof
14-O-{[(1R, 2R, 3R/S)-3-(Formyl-hydroxy-amino)-2-hydroxy-cyclohexylsulfanyl]-acetyl}-mutilin and the (1S, 2S, 3R/S) diastereomer thereof
14-O-{[(1R, 2R, 5S)- 2-Hydroxy-5-methylamino-cyclohexylsulfanyl]-acetyl}-mutilin and the (1S, 2S, 5R) diastereomer thereof
14-O-{[(1R, 2R, 5S)-5-Allylamino-2-hydroxy-cyclohexylsulfanyl]-acetyl}-mutilin and the (1S, 2S, 5R) diastereomer thereof
14-O-{[(1R, 2R, 5S)-2-Hydroxy-5-(2-methoxy-ethylamino)-cyclohexylsulfanyl]-acetyl}-mutilin and the (1S, 2S, 5R) diastereomer thereof
14-O-{[(1R, 2R, 4R^{∗})-2-Hydroxy-4-(2-hydroxy-ethylamino)-cyclohexylsulfanyl]-acetyl}-mutilin and the (1S, 2S, 4S^{∗}) diastereomer thereof
14-O-{[(1R, 2R, 4R^{∗})-4-Cyclohexylamino-2-hydroxy-cyclohexylsulfanyl]-acetyl}-mutilin and the (1S, 2S, 4S^{∗}) diastereomer thereof
14-O-{[(1R, 2R, 4R^{∗})-4-Cyclopropylamino-2-hydroxy-cyclohexylsulfanyl]-acetyl}-mutilin and the (1S, 2S, 4S^{∗}) diastereomer thereof
14-O-{[(1R, 2R, 5S^{∗})-4-Cyclopropylamino-2-hydroxy-cyclohexylsulfanyl]-acetyl}-mutilin and the (1S, 2S, 5R^{∗}) diastereomer thereof
14-O-{[(1R, 2R, 4S^{∗})-4-Cyclopropylamino-2-hydroxy-cyclohexylsulfanyl]-acetyl}-mutilin and the (1S, 2S, 4R^{∗}) diastereomer thereof
14-O-{[(1R, 2R, 5R^{∗})-2-Hydroxy-5-morpholin-4-yl-cyclohexylsulfanyl]-acetyl}-mutilin and the (1S, 2S, 5S^{∗}) diastereomer thereof
14-O-{[(1R, 2R, 5S^{∗})-2-Hydroxy-5-morpholin-4-yl-cyclohexylsulfanyl]-acetyl}-mutilin and the (1S, 2S, 5R^{∗}) diastereomer thereof
14-O-{[(1R, 2R, 5S)-5-Amino-2-hydroxy-cyclohexylsulfanyl]-acetyl}-19,20-dihydro-mutilin and the (1S, 2S, 5R) diastereomer thereof
14-O-{[(1R, 2R, 5S)-5-Ethylamino-2-hydroxy-cyclohexylsulfanyl]-acetyl}-19,20-dihydro-mutilin and the (1S, 2S, 5R) diastereomer thereof
14-O-{[(1R, 2R, 5R)-5-Amino-2-hydroxy-cyclohexylsulfanyl]-acetyl}-19,20-dihydro-mutilin and the (1S, 2S, 5S) diastereomer thereof
14-O-{[(1R, 2R)-4-Aminomethyl-2-hydroxy-cyclohexylsulfanyl]-acetyl}-mutilin and the (1S, 2S) diastereomers thereof
14-O-{[5-Amino-2-chloro-cyclohexylsulfanyl]-acetyl}-mutilin
14-O-{[4-Amino-2-chloro-cyclohexylsulfanyl]-acetyl}-mutilin
14-O-[(4-Amino-1-hydroxy-cyclohexylmethylsulfanyl)-acetyl]-mutilin
14-O-{[(1R, 2R)-2-Hydroxy-5-(3-methylamino-propyl)-cyclohexylsulfanyl]-acetyl}-mutilin and the (1S, 2S) diastereomer thereof
14-O-{[(1R, 2R)-2-Hydroxy-4-(3-methylamino-propyl)-cyclohexylsulfanyl]-acetyl}-mutilin and the (1S, 2S) diastereomer thereof
14-O-{[(1R, 2R)-5-(3-Amino-propyl)-2-hydroxy-cyclohexylsulfanyl]-acetyl}-mutilin and the (1S, 2S) diastereomer thereof
14-O-{[(1R, 2R)-4-(3-Amino-propyl)-2-hydroxy-cyclohexylsulfanyl]-acetyl}-mutilin and the (1S, 2S) diastereomer thereof
14-O-{[(6R, 8R)-8-Amino-1,4-dioxa-spiro[4.5]dec-6-ylsulfanyl]-acetyl}-mutilin and the (6S, 8S) diastereomer thereof
14-O-{[4-Amino-2-methoxy-cyclohexylsulfanyl]-acetyl}-mutilin
and
14-O-{[5-Amino-2-methoxy-cyclohexylsulfanyl]-acetyl}-mutilin.

4. A compound for use according to any of claims 1 to 3, wherein the compound is Lefamulin.

5. A compound for use according to any of claims 1 to 4, wherein the compound is in the form of a salt and/or a solvate.

6. A compound for use according to any of claims 1 to 5, wherein the compound is Lefamulin in the form as Lefamulin acetate salt.

7. A compound for use according to any of the preceding claims, wherein the bacterial infection is mediated by a subspecies of *F*. *tularensis* selected from the group of *F*. *tularensis* subsp. *tularensis* (Type A), *F*. *tularensis* subsp. *holarctica* (Type B), and *F*. *tularensis* subsp. *novicida.*

8. A compound for use according to any of the preceding claims, wherein the bacterial infection is tularemia.

9. A compound for use according to any of the preceding claims, wherein the compound is used for post-exposure prophylaxis (PEP).

10. A compound for use according to any of the preceding claims, wherein the compound is administered orally.

11. A method of treatment or prevention of a bacterial infection mediated by *F*. *tularensis* comprising administering a compound as defined in any of claims 1 to 6, in particular Lefamulin, or a pharmaceutically acceptable salt, solvate, ester of metabolite thereof to a subject in need of such treatment.

12. A method according to claim 11, wherein the subject is a human.

13. A method according to claim 11 or 12, wherein the bacterial infection is mediated by a subspecies of *F*. *tularensis* selected from the group of *F*. *tularensis* subsp. *tularensis* (Type A), *F*. *tularensis* subsp. *holarctica* (Type B), and *F*. *tularensis* subsp. *novicida.*

14. A method according to any one of claims 11 to 13, wherein the bacterial infection is tularemia.

15. A method according to any one of claims 11 to 14, wherein the method is for post-exposure prophylaxis (PEP).
